# EUROPEAN PATENT APPLICATION

(11) **EP 4 534 082 A1**
(43) Date of publication of application: **09.04.2025**
(21) Application number: 23835574.7
(22) Date of filing: 05.07.2023
(51) Int. Cl.: A61K 31/135, A61K 9/08, A61K 47/18, A61P 27/02

(54) **OPHTHALMIC COMPOSITION**

(30) Priority: 06.07.2022 JP 2022109258
(71) Applicant: Rohto Pharmaceutical Co., Ltd., Osaka-shi, Osaka 544-8666 (JP)
(72) Inventor: OGAWA Yoko, Osaka-shi, Osaka 544-8666 (JP)
(74) Representative: Mewburn Ellis LLP
(86) International application number: PCT/JP2023/024976
(87) International publication number: WO 2024/010043

(57) **Abstract**

The present invention relates to an ophthalmic composition containing tramadol or a salt thereof, in which the ophthalmic composition does not substantially contain a preservative.

## Description

### Technical Field

The present invention relates to an ophthalmic composition.

### Background Art

In general, a plurality of doses (multi-dose) of collyrium contain a preservative having an action of inhibiting the growth of microorganisms, such as bacteria and fungi, in preparation for when the collyrium is contaminated with the microorganisms. On the other hand, the preservative also contains cytotoxic components, and it has been reported that frequent or long-term use of collyrium containing a preservative causes corneal epithelial disorders. Therefore, there is a need for a prescription design of collyrium that inhibits the growth of microorganisms and reduces a risk of corneal epithelial disorders without using a preservative.

As a prescription design of such preservative-free collyrium, a technique of compounding a zinc salt such as zinc chloride to an ophthalmic composition has been reported (for example, Patent Literature 1).

### Citation List

### Patent Literature

Patent Literature 1: Japanese Unexamined Patent Publication No. 2010-504358

### Summary of Invention

### Technical Problem

An object of the present invention is to provide a novel ophthalmic composition that is preservative-free and has excellent preservative efficacy, and/or a novel ophthalmic composition having further enhanced preservative efficacy while containing a preservative.

### Solution to Problem

As a result of intensive studies to solve the above problems, the present inventors have found that when tramadol, which is a non-narcotic analgesic classified as a weak opioid and is used as a systemic analgesic for cancer pain, is compounded to an ophthalmic composition, unexpectedly, the preservative efficacy of the ophthalmic composition is remarkably enhanced. The present invention is based on this finding and provides the following inventions.
[1] An ophthalmic composition comprising tramadol or a salt thereof, wherein the ophthalmic composition does not substantially contain a preservative.
[2] An ophthalmic composition comprising tramadol or a salt thereof stored in a multi-dose container, wherein the tramadol or the salt thereof exhibits a preservative action, and an ophthalmic composition containing the same components as those of the ophthalmic composition in the same amount except that tramadol or a salt thereof is not contained does not exhibit a preservative action (with the proviso that an ophthalmic composition containing a preservative is excluded).
[3] An ophthalmic composition comprising tramadol or a salt thereof stored in a multi-dose container, wherein the tramadol or the salt thereof exhibits a preservative action, and exhibits a higher preservative action than an ophthalmic composition containing the same components as those of the ophthalmic composition in the same amount except that tramadol or a salt thereof is not contained.
[4] The ophthalmic composition according to any of [1] to [3], wherein a content of the tramadol or the salt thereof is 0.01 w/v% to 10 w/v% based on a total amount of the ophthalmic composition.
[5] The ophthalmic composition according to any one of [1] to [4], further comprising a buffer.
[6] A method for enhancing preservative efficacy of an ophthalmic composition that does not contain a preservative, the method comprising compounding tramadol or a salt thereof to the ophthalmic composition.

### Advantageous Effects of Invention

According to the present invention, it is possible to provide a novel ophthalmic composition that is preservative-free and has excellent preservative efficacy, and/or a novel ophthalmic composition having further enhanced preservative efficacy while containing a preservative.

### Description of Embodiments

Hereinafter, embodiments for carrying out the present invention will be described in detail. However, the present invention is not limited to the following embodiments.

In the present specification, a unit "%" of a content means "w/v%" and is synonymous with "g/100 mL" unless otherwise specified.

### [1. Ophthalmic Composition]

An ophthalmic composition according to the present embodiment contains (A) tramadol or a salt thereof (simply "component (A)").

### [Component (A)]

Tramadol is a known compound represented by the following formula: 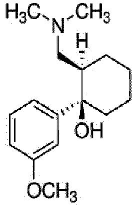 Note that, although the formula represents one of enantiomers for convenience, other enantiomers are also included in the present invention.

The salt of tramadol is not particularly limited as long as it is medicinally, pharmacologically (pharmaceutically), or physiologically acceptable. Specific examples of such a salt include a salt with an inorganic acid, a salt with an organic acid, a salt with an inorganic base, a salt with an organic base, a salt with an acidic amino acid, and a salt with a basic amino acid.

Examples of the salt with an inorganic acid include salts with hydrochloric acid, hydrobromic acid, sulfuric acid, nitric acid, phosphoric acid, and the like. Examples of the salt with an organic acid include salts with acetic acid, succinic acid, fumaric acid, maleic acid, tartaric acid, citric acid, lactic acid, stearic acid, benzoic acid, methanesulfonic acid (mesylic acid), ethanesulfonic acid, p-toluenesulfonic acid, and the like. Examples of the salt with an inorganic base include an alkali metal salt such as a sodium salt or a potassium salt, an alkaline earth metal salt such as a calcium salt or a magnesium salt, an aluminum salt, and an ammonium salt. Examples of the salt with an organic base include salts with diethylamine, diethanolamine, meglumine, N,N-dibenzylethylenediamine, and the like. Examples of the salt with an acidic amino acid include salts with aspartic acid, glutamic acid, and the like. Examples of the salt with a basic amino acid include salts with arginine, lysine, ornithine, and the like. As the salt of tramadol, a salt with an inorganic acid is preferable, and hydrochloride is more preferable.

The ophthalmic composition according to the present embodiment contains tramadol or a salt thereof as an active ingredient, and can be used for suppressing pain, for example.

A content of the component (A) in the ophthalmic composition according to the present embodiment is not particularly limited, and is appropriately set according to the type and content of other compounding ingredients, preparation form, and the like. The content of the component (A) may be 0.5 w/v% or more, 1 w/v% or more, 2 w/v% or more, or 3 w/v% or more, or may be 0.01 w/v% to 10 w/v%, 0.05 w/v% to 5 w/v%, 0.1 w/v% to 4 w/v%, or 1 w/v% to 3 w/v%, based on a total amount of the ophthalmic composition according to the present embodiment, from the viewpoint of more remarkably achieving the effect according to the present invention.

The ophthalmic composition according to the present embodiment may not substantially contain a preservative. In the present specification, "preservative" means a component exhibiting a preservative action. In the present specification, "does not substantially contain a preservative" means that no preservative is contained at all or a preservative is contained in an amount less than that required for exhibiting a preservative action. In the present specification, "exhibits a preservative action" means that an ophthalmic composition containing the substance meets the criteria in the preservative efficacy test method defined in the Japanese Pharmacopoeia 18th Edition. In the present specification, "high preservative efficacy" means that the preservative efficacy according to the preservative efficacy test method defined in the Japanese Pharmacopoeia 18th Edition is improved.

Examples of the preservative include alkylpolyaminoethylglycines, a quaternary ammonium salt (for example, benzalkonium chloride, benzethonium chloride, or the like), polidronium chloride, sodium benzoate, sodium dehydroacetate, methyl paraoxybenzoate, ethyl paraoxybenzoate, propyl paraoxybenzoate, butyl paraoxybenzoate, oxyquinoline sulfate, and a biguanide compound (specifically, polyhexanide hydrochloride (polyhexamethylene biguanide), chlorhexidine gluconate, alexidine, or the like). Since the ophthalmic composition according to the present embodiment can more remarkably exhibit the effect of the present invention, it is preferable that the ophthalmic composition does not contain a preservative at all. Note that metal salts such as a zinc salt (zinc chloride or the like) and a silver salt (silver nitrate or the like) are not included in the "preservative" in the present specification.

### [Buffer]

The ophthalmic composition according to the present embodiment preferably further contains a buffer. When the ophthalmic composition further contains a buffer, the effect of the present invention is more remarkably exhibited. The buffer is not particularly limited as long as it is medicinally, pharmacologically (pharmaceutically), or physiologically acceptable. Examples of the buffer include an inorganic buffer which is a buffer derived from an inorganic acid, and an organic buffer which is a buffer derived from an organic acid or an organic base.

Examples of the inorganic buffer include a boric acid buffer, a phosphoric acid buffer, and a carbonic acid buffer. Examples of the boric acid buffer include boric acid or a salt thereof (alkali metal borate, alkaline earth metal borate, or the like). Examples of the phosphoric acid buffer include phosphoric acid or a salt thereof (alkali metal phosphate, alkaline earth metal phosphate, or the like). Examples of the carbonic acid buffer include carbonic acid or a salt thereof (alkali metal carbonate, alkaline earth metal carbonate, or the like). In addition, as the boric acid buffer, the phosphoric acid buffer, or the carbonic acid buffer, a hydrate of boric acid, phosphoric acid, or carbonic acid may be used. As a more specific example, the boric acid buffer includes boric acid or a salt thereof (sodium borate, potassium tetraborate, potassium metaborate, ammonium borate, borax, or the like); the phosphoric acid buffer includes phosphoric acid or a salt thereof (disodium hydrogen phosphate, sodium dihydrogen phosphate, potassium dihydrogen phosphate, trisodium phosphate, tripotassium phosphate, calcium monohydrogen phosphate, calcium dihydrogen phosphate, or the like); and the carbonic acid buffer includes carbonic acid or a salt thereof (sodium bicarbonate, sodium carbonate, ammonium carbonate, potassium carbonate, calcium carbonate, potassium bicarbonate, magnesium carbonate, or the like).

Examples of the organic buffer include a citric acid buffer, an acetic acid buffer, a lactic acid buffer, a succinic acid buffer, a Tris buffer, and an AMPD buffer. Examples of the citric acid buffer include citric acid or a salt thereof (alkali metal citrate, alkaline earth metal citrate, or the like). Examples of the acetic acid buffer include acetic acid or a salt thereof (alkali metal acetate, alkaline earth metal acetate, or the like). Examples of the lactic acid buffer include lactic acid and a salt thereof (alkali metal lactate, alkaline earth metal lactate, or the like). Examples of the succinic acid buffer include succinic acid or a salt thereof (alkali metal succinate or the like). In addition, as the citric acid buffer, the acetic acid buffer, the lactic acid buffer, or the succinic acid buffer, a hydrate of citric acid, acetic acid, lactic acid, or succinic acid may be used. As a more specific example, the citric acid buffer includes citric acid or a salt thereof (sodium citrate, potassium citrate, calcium citrate, sodium dihydrogen citrate, disodium citrate, or the like); the acetic acid buffer includes acetic acid or a salt thereof (ammonium acetate, sodium acetate, potassium acetate, calcium acetate, or the like); the lactic acid buffer includes lactic acid or a salt thereof (sodium lactate, potassium lactate, calcium lactate, or the like); and the succinic acid buffer includes succinic acid or a salt thereof (monosodium succinate, disodium succinate, or the like). Examples of the Tris buffer include trometamol or a salt thereof (trometamol hydrochloride or the like). Examples of the AMPD buffer include 2-amino-2-methyl-1,3-propanediol or a salt thereof.

As the buffer, a boric acid buffer, a phosphoric acid buffer, and a citric acid buffer are preferable, a boric acid buffer and a phosphoric acid buffer are more preferable, and boric acid or a salt thereof and phosphoric acid or a salt thereof are still more preferable.

A commercially available buffer may be used. The buffers may be used alone or in combination of two or more types thereof.

A content of the buffer in the ophthalmic composition according to the present embodiment is not particularly limited, and is appropriately set according to the type of the buffer, the type and content of other compounding ingredients, the use and preparation form of the ophthalmic composition, and the like. The content of the buffer is, for example, preferably 0.01 w/v% to 10 w/v%, more preferably 0.05 w/v% to 5 w/v%, and still more preferably 0.1 w/v% to 3 w/v%, based on the total amount of the ophthalmic composition, from the viewpoint of more remarkably exhibiting the effect of the present invention. When the buffer is a boric acid buffer, the content is preferably 0.01 w/v% to 10 w/v%, more preferably 0.05 w/v% to 5 w/v%, still more preferably 0.1 w/v% to 3 w/v%, and particularly preferably 0.5 w/v% to 2.0 w/v%. When the buffer is a citric acid buffer or a phosphoric acid buffer, the content is preferably 0.01 w/v% to 10 w/v%, more preferably 0.05 w/v% to 5 w/v%, still more preferably 0.1 w/v% to 3 w/v%, still more preferably 0.1 w/v% to 1 w/v%, and particularly preferably 0.1 w/v% to 0.3 w/v%.

A content ratio of the buffer to the component (A) in the ophthalmic composition according to the present embodiment is not particularly limited, and is appropriately set according to the types of the component (A) and the buffer, the type and content of other compounding ingredients, the use and preparation form of the ophthalmic composition, and the like. From the viewpoint of further enhancing the effect of the present invention, the content ratio of the buffer to the component (A) may be, for example, 0.001 to 1,000 parts by mass, 0.01 to 100 parts by mass, or 0.025 to 30 parts by mass, with respect to 1 part by mass of the total content of the component (A) contained in the ophthalmic composition according to the present embodiment.

### [Inorganic Salt]

The ophthalmic composition according to the present embodiment may further contain an inorganic salt. When the ophthalmic composition further contains an inorganic salt, the effect of the present invention is more remarkably exhibited. The inorganic salt is not particularly limited as long as it is medicinally, pharmacologically (pharmaceutically), or physiologically acceptable.

Examples of the inorganic salt include chloride salts such as sodium chloride, potassium chloride, calcium chloride, and magnesium chloride. As the inorganic salt, sodium chloride and potassium chloride are preferable.

A commercially available inorganic salt may be used. The inorganic salts may be used alone or in combination of two or more types thereof.

A content of the inorganic salt in the aqueous composition according to the present embodiment is not particularly limited, and is appropriately set according to the type of the inorganic salt, the type and content of other compounding ingredients, the use and preparation form of the aqueous composition, and the like. The content of the inorganic salt is, for example, preferably 0.00001 w/v% to 3 w/v%, more preferably 0.0001 w/v% to 2 w/v%, and still more preferably 0.001 w/v% to 1.5 w/v%, based on the total amount of the ophthalmic composition, from the viewpoint of more remarkably exhibiting the effect of the present invention.

A pH of the ophthalmic composition according to the present embodiment is not particularly limited as long as it is within a medicinally, pharmacologically (pharmaceutically), or physiologically acceptable range. The pH of the ophthalmic composition according to the present embodiment may be, for example, 4.5 to 7.5, preferably 5.0 to 7.0, and more preferably 5.5 to 6.5.

The ophthalmic composition according to the present embodiment can be adjusted to an osmotic pressure ratio within a range acceptable to a living body as necessary. An appropriate osmotic pressure ratio can be appropriately set according to the use, preparation form, use method, and the like of the ophthalmic composition, and can be, for example, 0.4 to 5.0. The osmotic pressure ratio is defined as a ratio of an osmotic pressure of a sample to 286 mOsm (an osmotic pressure of a 0.9 w/v% aqueous sodium chloride solution) based on the Japanese Pharmacopoeia 18th Edition, and the osmotic pressure is measured with reference to the osmotic measurement method (freezing point depression method) described in the Japanese Pharmacopoeia. Note that, as the standard solution for measuring an osmotic pressure ratio (0.9 w/v% aqueous sodium chloride solution), sodium chloride (Japanese Pharmacopoeia standard reagent) is dried at 500 to 650°C for 40 to 50 minutes and then allowed to cool in a desiccator (silica gel), and 0.900 g of the dried solution is accurately weighed and dissolved in purified water to prepare exactly 100 mL of a solution, or a commercially available standard solution for measuring an osmotic pressure ratio (0.9 w/v% aqueous sodium chloride solution) can be used.

A viscosity of the ophthalmic composition according to the present embodiment is not particularly limited as long as it is within a medicinally, pharmacologically (pharmaceutically), or physiologically acceptable range. As the viscosity of the ophthalmic composition according to the present embodiment, for example, the viscosity at 20°C measured with a rotational viscometer (TV-20 type viscometer, manufactured by Toki Sangyo Co., Ltd., rotor; 1° 34' × R24) may be 1 to 10,000 mPa·s.

The ophthalmic composition according to the present embodiment can be prepared, for example, by adding and mixing the component (A) and, if necessary, other contained components so as to have a desired content. Specifically, for example, the ophthalmic composition can be prepared by dissolving or suspending the above components in purified water, and subjecting the solution to sterilization treatment by filtration sterilization or the like.

The ophthalmic composition according to the present embodiment can be in various dosage forms depending on the purpose, and examples thereof include a liquid preparation, a gel preparation, and a semi-solid preparation (ointment or the like).

When the ophthalmic composition according to the present embodiment is a liquid preparation, the ophthalmic composition for example, collyrium (also referred to as an eye drop or an eye lotion, and in addition, collyrium includes collyrium that can be applied while wearing contact lenses), an artificial tear solution, an eye wash (also called an eye wash solution or an eye wash drug, and in addition, the eye wash includes an eye wash that can wash the eye while wearing contact lenses). Note that the "contact lens" includes a hard contact lens and a soft contact lens (both ionic and nonionic lenses are encompassed, and both a silicone hydrogel contact lens and a non-silicone hydrogel contact lens are encompassed).

The ophthalmic composition according to the present embodiment is preferably collyrium (collyrium that can be instilled while wearing contact lenses) because the effect of the present invention can be more remarkably exhibited. When the ophthalmic composition according to the present embodiment is collyrium, the dosage and dose are not particularly limited as long as it is effective and has few side effects. For example, in the case of adults (15 years old or older) and children of 7 years old or older, there is a method of instilling 1 to 3 drops, 1 or 2 drops, or 2 or 3 drops at a time 1 to 4 times or 5 or 6 times a day and using the ophthalmic composition.

The ophthalmic composition according to the present embodiment is provided in an arbitrary container. The container for storing the ophthalmic composition according to the present embodiment is not particularly limited, and may be formed of, for example, glass or plastic. The container is preferably formed of plastic. Examples of the plastic include polyethylene terephthalate (PET), polybutylene terephthalate (PBT), polyethylene naphthalate, polyarylate, polycarbonate, polyethylene (PE, high density polyethylene (HDPE), low density polyethylene (LDPE), or linear low density polyethylene (LLDPE)), polypropylene (PP), polystyrene (PS), acrylonitrile butadiene styrene (ABS), polymethylpentene (PMP), polyimide (PI), a cyclic olefin polymer (COP), a cyclic olefin copolymer (COC), a copolymer of monomers constituting these polymers, and a mixture of two or more of these polymers.

The container for storing the ophthalmic composition may be a container commonly used in the field of ophthalmology, and specifically, may be, for example, an eye drop container or an eye wash solution container. The type of the container is preferably an eye drop container.

The shape and volume of the container are not particularly limited, and may be appropriately set according to the use. In addition, the container may be a container (multi-dose container) in which a plurality of doses of the ophthalmic composition are stored, or may be a container (unit dose container) in which a single dose of the ophthalmic composition is stored.

When the container is a multi-dose container, for example, a volume may be 1.5 to 7.5 mL, 2 to 6 mL, or 2.5 to 5.0 mL. In addition, when the container is a unit dose container, a volume may be, for example, 0.1 to 1.0 mL, 0.2 to 0.9 mL, or 0.3 to 0.8 mL.

The ophthalmic composition according to the present embodiment can also be provided as a packaged ophthalmic composition. The present invention can also be regarded as an ophthalmic product (collyrium or the like) storing the ophthalmic composition of the present invention in a container.

### [2. Method for Enhancing Preservative Efficacy of Ophthalmic Composition]

The ophthalmic composition according to the present embodiment contains (A) tramadol or a salt thereof, such that the preservative efficacy is enhanced while being preservative-free. Therefore, as an embodiment of the present invention, there is provided a method for enhancing preservative efficacy of an ophthalmic composition that does not contain a preservative, the method including compounding (A) tramadol or a salt thereof to the ophthalmic composition.

The type and content of the component (A), the type and content of other components, and the preparation form and use of the ophthalmic composition in the method are as described in [1. Ophthalmic Composition].

### Examples

Hereinafter, the present invention will be specifically described based on test examples, but the present invention is not limited thereto. In addition, unless otherwise specified, a unit of each component in the table is w/v%.

### [Test Example 1: Preservative Efficacy Test]

Ophthalmic compositions having the compositions shown in Table 1 were prepared according to a conventional method. Each of the prepared ophthalmic compositions was filtered through a 0.2 µm membrane filter and sterilized. The preservative efficacy of each ophthalmic composition was evaluated according to the preservative efficacy test method specified in the Japanese Pharmacopoeia 18th Edition. Specifically, *S. aureus*, *E. coli*, and *Pseudomonas aeruginosa* were inoculated onto a surface of a soybean-casein digest agar medium, and cultured at 30 to 35°C for 24 hours. The cultured cells were aseptically collected with a platinum loop and suspended in an appropriate amount of sterile physiological saline to prepare a bacterial suspension containing viable cells at about 1 × 10⁷ CFU/mL. The bacterial suspension was added to each preparation so as to be about 1 × 10⁵ CFU/mL, and then the preparation was allowed to stand at 20 to 25°C for 7 days. Thereafter, bacteria were recovered according to the agar mixing method and were allowed to stand at 30 to 35°C for 2 or 3 days, and then the number of viable bacteria per 1 mL of each ophthalmic composition was measured. Log reduction was calculated based on the measured value, and the preservative efficacy was determined according to the preservative efficacy test method (preparation category IA). The results are shown in Table 1.

**[Table 1]**

| | Example 1 | Comparative Example 1 | Example 2 | Comparative Example 2 | Example 3 | Comparative Example 3 | Example 4 | Comparative Example 4 |
|---|---|---|---|---|---|---|---|---|
| Boric acid | 1 | 1 | - | - | - | - | 2 | 2 |
| Borax | 0.005 | 0.005 | - | - | - | - | 0.02 | 0.02 |
| Sodium dihydrogen phosphate hydrate | - | - | 0.3 | 0.3 | - | - | - | - |
| Sodium hydrogen phosphate hydrate | - | - | 0.1 | 0.1 | - | - | - | - |
| Sodium chloride | - | - | 0.2 | 0.2 | - | - | - | - |
| Tramadol hydrochloride | 3 | - | 3 | - | 3 | - | 1 | - |
| Dilute hydrochloric acid | Appropriate amount | Appropriate amount | Appropriate amount | Appropriate amount | Appropriate amount | Appropriate amount | Appropriate amount | Appropriate amount |
| Sodium hydroxide | Appropriate amount | Appropriate amount | Appropriate amount | Appropriate amount | Appropriate amount | Appropriate amount | Appropriate amount | Appropriate amount |
| Purified water | Remaining amount | Remaining amount | Remaining amount | Remaining amount | Remaining amount | Remaining amount | Remaining amount | Remaining amount |
| Total amount | 100 mL | 100 mL | 100 mL | 100 mL | 100 mL | 100 mL | 100 mL | 100 mL |
| pH | 6.0 | 6.0 | 6.0 | 6.0 | 6.0 | 6.0 | 6.0 | 6.0 |
| S.aureus | | | | | | | | |
| For 1 week | > 4.4 | 2.8 | 3.2 | 1.1 | > 4.4 | 4.4 | > 4.4 | 4.0 |
| For 2 weeks | > 4.4 | > 4.4 | > 4.4 | 4.0 | > 4.4 | > 4.4 | > 4.4 | > 4.4 |
| For 4 weeks | > 4.4 | > 4.4 | > 4.4 | > 4.4 | > 4.4 | > 4.4 | > 4.4 | > 4.4 |
| E.coli | | | | | | | | |
| For 1 week | 4.6 | 0.6 | 4.0 | 0.1 | > 4.6 | 0.0 | 1.2 | 0.5 |
| For 2 weeks | > 4.6 | 1.0 | > 4.6 | 0.3 | > 4.6 | 0.1 | 3.0 | 0.8 |
| For 4 weeks | > 4.6 | 2.4 | > 4.6 | 0.5 | > 4.6 | 0.2 | > 4.6 | 2.1 |
| P.aeruginosa | | | | | | | | |
| For 1 week | > 4.4 | 1.3 | > 4.4 | 0.4 | > 4.4 | 2.2 | 4.1 | 2.6 |
| For 2 weeks | > 4.4 | 2.5 | > 4.4 | 0.4 | > 4.4 | 2.6 | > 4.4 | 4.1 |
| For 4 weeks | > 4.4 | > 4.4 | > 4.4 | 0.4 | > 4.4 | 3.1 | > 4.4 | > 4.4 |
| Determination | Suitable | Unsuitable | Suitable | Unsuitable | Suitable | Unsuitable | Suitable | Unsuitable |

In Comparative Examples 1 to 4 in which tramadol hydrochloride was not compounded, the preservative efficacy was extremely low. On the other hand, in Examples 1 to 4 in which tramadol hydrochloride was compounded, it was confirmed that the preservative efficacy was remarkably enhanced as compared with Comparative Examples 1 to 4.

## Claims

1. An ophthalmic composition comprising tramadol or a salt thereof, wherein the ophthalmic composition does not substantially contain a preservative.

2. An ophthalmic composition comprising tramadol or a salt thereof stored in a multi-dose container, wherein the tramadol or the salt thereof exhibits a preservative action, and an ophthalmic composition containing the same components as those of the ophthalmic composition in the same amount except that tramadol or a salt thereof is not contained does not exhibit a preservative action (with the proviso that an ophthalmic composition containing a preservative is excluded).

3. An ophthalmic composition comprising tramadol or a salt thereof stored in a multi-dose container, wherein the tramadol or the salt thereof exhibits a preservative action, and exhibits a higher preservative action than an ophthalmic composition containing the same components as those of the ophthalmic composition in the same amount except that tramadol or a salt thereof is not contained.

4. The ophthalmic composition according to any of claims 1 to 3, wherein a content of the tramadol or the salt thereof is 0.01 w/v% to 10 w/v% based on a total amount of the ophthalmic composition.

5. The ophthalmic composition according to any one of claims 1 to 3, further comprising a buffer.

6. A method for enhancing preservative efficacy of an ophthalmic composition, the method comprising compounding tramadol or a salt thereof to the ophthalmic composition.
